# EUROPEAN PATENT APPLICATION

(11) **EP 4 394 031 A1**
(43) Date of publication of application: **03.07.2024**
(21) Application number: 23856254.0
(22) Date of filing: 27.06.2023
(51) Int. Cl.: C12N 5/0775, A61K 35/28, A61P 37/02, A61P 29/00

(54) **MESENCHYMAL STEM CELL AND EXOSOME THEREOF OBTAINED BY TREATMENT OF AT LEAST TWO CYTOKINES OF IL4, IL21, AND IL27, AND USE THEREOF**

(30) Priority: 24.08.2022 CN 202211044883
(71) Applicant: Shenzhen Beike Biotechnology Co., Ltd., Shenzhen, Guangdong 518000 (CN)
(72) Inventor: LIAO, Yan, Shenzhen, Guangdong 518000 (CN); FU, Zeqin, Shenzhen, Guangdong 518000 (CN); LI, Duanduan, Shenzhen, Guangdong 518000 (CN); YANG, Yulin, Shenzhen, Guangdong 518000 (CN); HUANG, Yinfu, Shenzhen, Guangdong 518000 (CN); CAI, Cheguo, Shenzhen, Guangdong 518000 (CN); HU, Junyuan, Shenzhen, Guangdong 518000 (CN)
(74) Representative: Zaboliene, Reda
(86) International application number: PCT/CN2023/102557
(87) International publication number: WO 2024/041162

(57) **Abstract**

The present invention discloses a mesenchymal stem cell treated by at least two of cytokines IL4, IL21, and IL27, an exosome and use thereof. The present invention relates to the technical field of biomedicine; the mesenchymal stem cell and exosome thereof are obtained after the mesenchymal stem cell is pretreated with a cytokine composition. The mesenchymal stem cell is pretreated in a way of: treating and stimulating the mesenchymal stem cell by stages with a complete medium containing the cytokine composition to obtain a capacitated mesenchymal stem cell; the exosome of the mesenchymal stem cell is pretreated in a way of: treating and stimulating the mesenchymal stem cell by stages with a complete medium containing the cytokine composition, and replacing with a basal medium for starvation treatment. The mesenchymal stem cell obtained after being pretreated with the cytokine composition and an exosome thereof provided in the present invention can exert a stronger immune modulating effect and thus, can be used for the treatment of immune diseases better.

## Description

### FIELD OF TECHNOLOGY

The present invention relates to the technical field of biomedicine, and in particular to a mesenchymal stem cell treated by at least two of cytokines IL4, IL21, and IL27, an exosome and use thereof.

### BACKGROUND

Refractory systemic lupus erythematosus (SLE) is a kind of autoimmune disease caused by multiple factors, and preferably occurs in 20 to 40-week old women, and features slow onset and a variety of clinical manifestations. Autoantibodies produced by patients due to the disorder existing in their autologous cells and humoral immune function, will involve many visceral organs of the whole body, such as, skin, joint, kidney, and nervous system. Common symptoms include arthritis, fever, chest pain, alopecia, lymphadenectasis, and facial erythema. The cause of SLE has been not clear. Currently, SLE is believed to be possibly caused by the interaction of genetic factors, environmental factors, endocrinal factors. In China, the average incidence of the SLE population is 30-70 people/100,000 people having a male-female ratio of 1:11.9 and an average age of attack of 30.7 years old. Once the onset of SLE, lethality and disability rate are higher to the patients, which thus will cause certain financial and psychological burden to the patients and their families. In the clinic treatment for refractory SLE, there is no completely cured case through conventional therapies; the existing traditional therapies will gradually lose their due therapeutic effects with the course of disease. Somatic function thus will be disintegrated gradually under the long-term chronic inflammatory reaction, which will greatly affect the living quality of the patients.

Mesenchymal stem cells are a category of pluripotent stem cells, and widely exist in various tissues and organs of our body, and function in tissue repair and immune modulation. Mesenchymal stem cells feature multi-directional differentiation potential, self-renewal, and fast multiplication rate *in vitro.* Their biological characteristics concerned extensively mainly lie in possessing lower immunogenicity and stronger immune modulating capacity. Therefore, mesenchymal stem cells in quantity have been applied to numerous clinical tests and favored by numerous specialists and scholars in the art. In the clinical tests with the application of mesenchymal stem cells, a large part of tests relate to the treatment of autoimmune diseases such as SLE, rheumatoid arthritis, psoriasis, and Crohn's disease, indicating that mesenchymal stem cells have better application contexts in the treatment of autoimmune diseases. Moreover, certain therapeutic effects have been also achieved. However, not all the patients participating in the clinical tests with the use of mesenchymal stem cells achieve a better therapeutic effect. There exists a certain individual difference in the treatment of autoimmune diseases via ordinary mesenchymal stem cells; therefore, often not all the subjects achieve a better expected efficacy, which is mainly caused by the heterogeneity of mesenchymal stem cells per se. Ordinary mesenchymal stem cells prepared for conventional clinic treatment are passaged and amplified for many times, but there are still cell subsets having various functional traits among the cell population. Mesenchymal stem cells mainly exert its immune modulating effect to autoimmune diseases by paracrine pathways. Because of cellular heterogeneity, during clinic treatment, ordinary mesenchymal stem cells need to be stimulated via *in vivo* microenvironment after being injected into the body, and then will gradually form cell subsets with uniform functions and similar characters, and finally exert the therapeutic effects. However, cells have a relatively fixed metabolic cycle after being injected into the body, and a large part of cells will be entrapped in lung and thus will not migrate around the whole body. Cells must receive an *in vivo* stimulation before come into play, which occupies most of time within the metabolic cycle; the time to exert therapeutic effect will be reduced greatly, thus resulting in different therapeutic effects among different patients.

Exosome is a category of small vesicles containing complex RNA, proteins or saccharides, and usually has a diameter range of 40-100 nm. Exosome will be secreted extracellularly in various cellular states. As a specifically secreted vesicle, exosome plays an important role in cell communication and intercellular interaction. Moreover, exosome only has a membrane structure and has no nuclear structure and thus, is less likely to cause the problem of safety, and has been widely concerned in clinical research and academic research. Exosome plays a paracrine role in mesenchymal stem cells and is of great significance in regulating body immunity. However, ordinary mesenchymal stem cells hardly perform reaction upon entering the body and thus, only secrete a very limited number of exosomes having an immune modulating effect. Therefore, there is a need for a technical way more such that mesenchymal stem cells are stimulated by the corresponding inflammatory environment signal to rapidly produce a large number of corresponding exosomes having corresponding modulating functions in the corresponding inflammatory environment.

To sump, solving the activization of mesenchymal stem cells is the key problem to improve/enhance the therapeutic effect of mesenchymal stem cells. The existing research proves that after receiving the stimulation of corresponding cytokine *in vitro,* mesenchymal stem cells will show the change in the corresponding biological characteristics, and will bring different effects based on the type, concentration, and degree of stimulation of the cytokine. Therefore, to provide a mesenchymal stem cell for use in the treatment of SLE and its exosome is of great significance.

### SUMMARY

In view of this, directed to the defects of the prior art, the present invention is mainly aimed at providing a mesenchymal stem cell treated by at least two of cytokines IL4, IL21, and IL27, an exosome and use thereof. The mesenchymal stem cell obtained after being pretreated by a cytokine composition and an exosome thereof provided in the present invention can exert a stronger immune modulating effect and thus, can be used for the treatment of immune diseases better.

To achieve the above object, the present invention adopts the following technical solution:

A mesenchymal stem cell treated by at least two of cytokines IL4, IL21, and IL27, an exosome and use thereof are provided; the mesenchymal stem cell and exosome thereof are obtained after the mesenchymal stem cell is pretreated with a cytokine composition. The cytokine composition includes at least two of cytokines IL4, IL21, and IL27. The mesenchymal stem cell is pretreated in a way of: treating and stimulating the mesenchymal stem cell by stages with a complete medium containing the cytokine composition by a large-scale bioreactor to obtain a capacitated mesenchymal stem cell. The exosome of the mesenchymal stem cell is pretreated in a way of: treating and stimulating the mesenchymal stem cell by stages with a complete medium containing the cytokine composition by a large-scale bioreactor, replacing with a basal medium for starvation treatment, collecting a cultural supernatant, and conducting gradient ultracentrifugation to obtain the exosome.

As a preferred embodiment, the way of treating and stimulating by stages is as follows: complete media containing different cytokine compositions having corresponding concentrations are added to a large-scale bioreactor to capacitate and stimulate the mesenchymal stem cell. The complete medium is a pooled serum substitute for any basal medium for culturing a mammalian cell.

As a preferred embodiment, the three cytokines IL4, IL21, and IL27 have concentration ranges of IL4:25-150 ng/ml:IL21:100-300 ng/ml, and IL27:50-250 ng/ml, respectively.

As a preferred embodiment, the cytokine composition includes IL4 and IL21; or, the cytokine composition includes IL21 and IL27; or, the cytokine composition includes IL4 and IL27.

As a preferred embodiment, the cytokine composition comprises the IL4 having a concentration of 30 ng/ml and the IL21 having a concentration of 200 ng/ml; or, the cytokine composition comprises the IL21 having a concentration of 150 ng/ml and the IL27 having a concentration of 200 ng/ml; or the cytokine composition comprises the IL4 having a concentration of 25 ng/ml and the IL27 having a concentration of 75 ng/ml.

As a preferred embodiment, contact culture is required in the pretreatment process of the mesenchymal stem cell by the cytokine composition for 16-36 h. The mesenchymal stem cell is pretreated with the IL4 in the cytokine composition containing IL4 and IL21 for 10 h and pretreated with the IL21 in the cytokine composition containing IL4 and IL21 for 12 h; the mesenchymal stem cell is pretreated with the IL21 in the cytokine composition containing IL21 and IL27 for 10 h and pretreated with the IL27 in the cytokine composition containing IL21 and IL27 for 12 h; and the mesenchymal stem cell is pretreated with the IL4 in the cytokine composition containing IL4 and IL27 for 6 h and pretreated with the IL27 in the cytokine composition containing IL4 and IL27 for 10 h.

As a preferred embodiment, the basal medium is used for starvation treatment for 24 h.

As a preferred embodiment, the mesenchymal stem cell not pretreated is derived from at least one of the group consisting of bone marrow, fat, placenta, umbilical cord, and dental pulp in a human tissue; and the exosome is secreted from the mesenchymal stem cell.

As a preferred embodiment, the mesenchymal stem cell treated by at least two of cytokines IL4, IL21, and IL27, and an exosome thereof have an enhanced anti-SLE curative activity.

Use of the mesenchymal stem cell treated by at least two of cytokines IL4, IL21, and IL27, and an exosome thereof is for use in the preparation of a medicament for treating a systemic and immune inflammation caused by SLE.

Compared with the prior art, the present invention has the obvious advantages and beneficial effects. Specifically, as can be seen from the above technical solution, the cytokine compositions provided by the present invention are screened out according to the expression characteristics of the cytokines in the *in vivo* inflammatory microenvironment during the course of the SLE patient, and can stimulate human mesenchymal stem cells to achieve better anti-inflammatory activity against SLE. Compared with the human mesenchymal stem cell obtained through conventional methods and its exosome, the mesenchymal stem cell and its exosome provided by the present invention can have better activity in the anti-inflammatory environment and thus, can be used for the treatment of immuno-inflammatory responses caused by SLE, thereby reducing the damage of inflammatory response on the systemic tissues and relieving the rate of disease progression, which is specifically embodied in: reducing a proliferation proportion of TIE lymphocytes, reducing a proliferation ratio of helper T cells, and up-regulating a proliferation ratio of regulatory T cells. Moreover, the mesenchymal stem cell cultured by the method of the present invention and the exosome thereof are superior to those cultured by a conventional method in the degree of protection for disease models.

To describe the structural features and efficacy of the present invention more clearly, the present invention will be described in detail hereafter with reference to the accompanying drawings and detailed embodiments.

### BRIEF DESCRIPTION OF THE DRAWINGS

FIG. 1 shows changes of IL4, IL21, and IL27 in serum *in vivo* of mice in a SLE model during the progression of disease according to the present invention;
FIG. 2 shows therapeutic effects of a human umbilical cord mesenchymal stem cell before and after being pretreated, and an exosome thereof, reflected on dsDNA and ANA of SLE mice serum according to the present invention;
FIG. 3 shows influences of the human umbilical cord mesenchymal stem cell before and after being pretreated, and an exosome thereof on biological characteristics according to the present invention (A denotes the expression of IDO in different groups; B denotes the expression of PGE2 in different groups; C denotes the expression of VEGF in different groups);
FIG. 4 shows influences on cytokines expressed by the human umbilical cord mesenchymal stem cell and an exosome thereof at different concentrations of cytokines according to the present invention (A denotes the expression of IDO in different groups; and B denotes the expression of PGE2 in different groups);
FIG. 5 shows influences on *in vitro* immunosuppression function of the human umbilical cord mesenchymal stem cell after being pretreated with different cytokine compositions and an exosome thereof according to the present invention;
FIG. 6 shows influences of the cytokine composition on *in vitro* immunosuppression function of the human umbilical cord mesenchymal stem cell and an exosome thereof treated at different treatment time according to the present invention;
FIG. 7 shows influences of the cytokine composition on *in vitro* immunosuppression function of the human umbilical cord mesenchymal stem cell and an exosome thereof treated at different concentrations according to the present invention; and
FIG. 8 shows curative effects of the human umbilical cord mesenchymal stem cell after being pretreated with different cytokine compositions and an exosome thereof under a SLE model according to the present invention.

### DESCRIPTION OF THE EMBODIMENTS

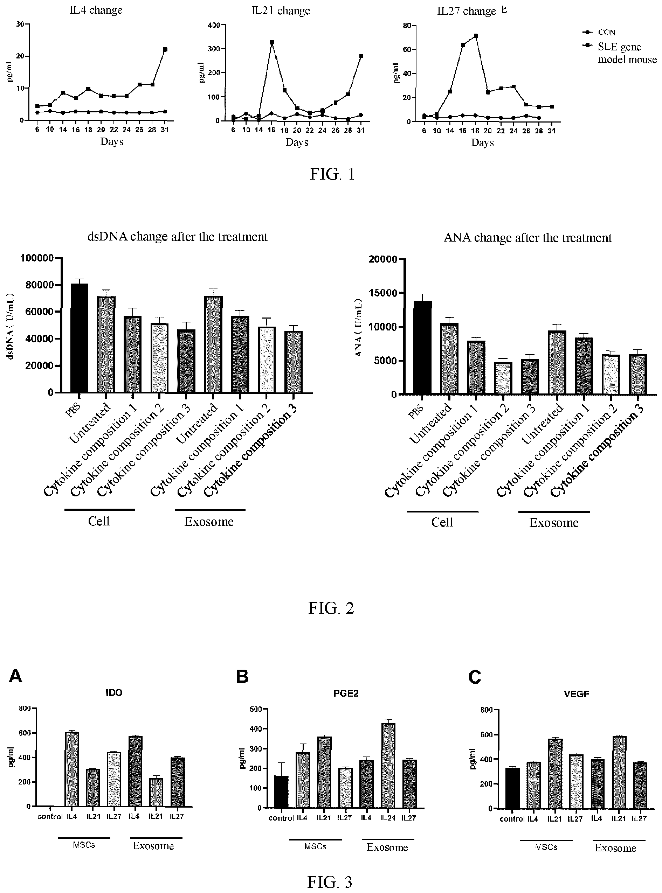

As shown in FIGS. 1-8, the present invention provides a mesenchymal stem cell treated by at least two of cytokines IL4, IL21, and IL27, an exosome and use thereof; the mesenchymal stem cell and exosome thereof are obtained after the mesenchymal stem cell is pretreated with a cytokine composition. The cytokine composition includes at least two of cytokines IL4, IL21, and IL27. The mesenchymal stem cell is pretreated in a way of: treating and stimulating the mesenchymal stem cell by stages with a complete medium containing the cytokine composition by a large-scale bioreactor to obtain a capacitated mesenchymal stem cell; the exosome of the capacitated mesenchymal stem cell is pretreated in a way of: treating and stimulating the mesenchymal stem cell by stages with a complete medium containing the cytokine composition by a large-scale bioreactor, replacing with a basal medium for starvation treatment, collecting a cultural supernatant, and conducting gradient ultracentrifugation to obtain the exosome.

As a preferred embodiment, the way of treating and stimulating by stages is as follows: a complete medium containing different cytokine compositions having corresponding concentrations is added to a large-scale bioreactor to capacitate and stimulate the mesenchymal stem cell. The complete medium is a pooled serum substitute for any basal medium for culturing a mammalian cell.

The basal medium may be a Dulbecco's Modified Eagle Medium (DMEM), a mixture of a DMEM with F12 (DMEM/F12), and RPMI 1640.

The three cytokines IL4, IL21, and IL27 have concentration ranges of IL4:25-150 ng/ml:IL21:100-300 ng/ml, and IL27:50-250 ng/ml, respectively.

The cytokine composition includes IL4 and IL21; or, the cytokine composition includes IL21 and IL27; or, the cytokine composition includes IL4 and IL27.

The cytokine composition includes the IL4 having a concentration of 30 ng/ml and the IL21 having a concentration of 200 ng/ml; or, the cytokine composition comprises the IL21 having a concentration of 150 ng/ml and the IL27 having a concentration of 200 ng/ml; or the cytokine composition comprises the IL4 having a concentration of 25 ng/ml and the IL27 having a concentration of 75 ng/ml.

Contact culture is required in the pretreatment process of the mesenchymal stem cell by the cytokine composition for 16-36 h. The mesenchymal stem cell is pretreated with the IL4 in the cytokine composition containing IL4 and IL21 for 10 h and pretreated with the IL21 in the cytokine composition containing IL4 and IL21 for 12 h; the mesenchymal stem cell is pretreated with the IL21 in the cytokine composition containing IL21 and IL27 for 10 h and pretreated with the IL27 in the cytokine composition containing IL21 and IL27 for 12 h; and the mesenchymal stem cell is pretreated with the IL4 in the cytokine composition containing IL4 and IL27 for 6 h and pretreated with the IL27 in the cytokine composition containing IL4 and IL27 for 10 h.

The basal medium is used for starvation treatment for 24 h.

The mesenchymal stem cell not pretreated is derived from at least one of the group consisting of bone marrow, fat, placenta, umbilical cord, and dental pulp in a human tissue; and the exosome is secreted from the mesenchymal stem cell.

The mesenchymal stem cell obtained after being treated with at least two of cytokines IL4, IL21, and IL27, and an exosome thereof have an enhanced anti-SLE curative activity.

Use of the mesenchymal stem cell obtained after being treated with at least two of cytokines IL4, IL21, and IL27, and an exosome thereof is for use in the preparation of a medicament for treating a systemic and immune inflammation caused by systemic lupus erythematosus (SLE).

In the present invention, the reagents and test materials used can be commercially available, of which biomaterials are derived from the followings:
Cytokines IL4, IL21, and IL27 are purchased from R&D Systems.

### Example I:

Preparation method of the human umbilical cord mesenchymal stem cell pretreated with a cytokine composition and an exosome thereof:
Firstly: preparation method of the human umbilical cord mesenchymal stem cell:
Isolation and culture of the human umbilical cord mesenchymal stem cell:
Under the supervision of the Medical Ethic Committee, a fresh umbilical cord was obtained from a healthy donor, and isolated with Wharton's jelly; the Wharton's jelly was cut into pieces, and an adherence method was used for cell culture; primary cells were obtained and amplified under a serum-free culture system. The serum-free culture system is: MSCNutristem^{®}XF Medium (BI, Israel) + 2% platelet lysate + MSCNutristem^{®}XF Supplement Mix(BI, Israel).

The primary cells were amplified and cryopreserved at the "P2 generation" as seed cells:
The "P2 generation" refers as follows: the primary cells were subjected to multiplication culture in a cell factory at 3000-5000 cells/m2 to accumulate a certain amount, then transferred into a cylindrical cell expansion bioreactor; the cells were mixed with a microcarrier for culture and amplified at a concentration of 10000-12000 cells/ml; nutrient solution was supplemented timely with a perfusion system, 24 h later after the culture, the cells got into the treatment stage of the cytokine composition.

Secondly: pretreatment of the human umbilical cord mesenchymal stem cell by a cytokine composition:
Different cytokines were added to cell media at the corresponding concentrations by stages based on the way of combinations to replace the original media gradually with the perfusion system. According to the corresponding sequence, each cytokine in the combinations was used to stimulate the human umbilical cord mesenchymal stem cell for a corresponding time, and then replaced with the next cytokine; or the basal medium was replaced for starvation treatment.

The "pretreatment" refers to the following process: during the culture process of the mesenchymal stem cell derived from human umbilical cord, a cell medium added with the cytokine composition containing at least two of cytokines IL4, IL21, and IL27 was subjected to contact culture with the mesenchymal stem cell derived from human umbilical cord.

The "basal medium" may be any commercially available cell medium suitable for mammal, such as DMEM, MEM, and DMEM/F12. MSCNutristem^{®}XF Medium (BI, Israel) was used in this example.

In the description, based on the pathological characteristics of SLE, the cytokine composition includes IL4 and IL21, called a cytokine composition 1; the cytokine composition includes IL21 and IL27, called a cytokine composition 2; the cytokine composition includes IL4 and IL27, called a cytokine composition 3. The above cytokines have concentration ranges of IL4:25-150 ng/ml, IL21:100-300 ng/ml, IL27:50-250 ng/ml.

In the description, time for the contact culture between the cell and the pretreatment medium containing the cytokine composition is as follows:
In the cytokine composition 1, the cell is pretreated with the IL4 for 10 h and pretreated with the IL21 for 12 h; in the cytokine composition 2, the cell is pretreated with the IL21 for 10 h and pretreated with the IL27 for 12 h; and in the cytokine composition 3, the cell is pretreated with the IL4 for 6 h and pretreated with the IL27 for 10 h.

In the description, the mesenchymal stem cell treated by the pretreatment medium containing the cytokine composition is still subjected to starvation treatment for 24 h before being digested, and prepared into a cell preparation, then stored and transported to a patient.

### Thirdly: preparation method of the exosome

Cultural supernatant after the starvation treatment was collected, and centrifuged at a centrifugal force of 200 g for 10 min to remove most of cell debris, afterwards, a hollow fiber column, as a filter carrier, was used for tangential flow concentration, and pressure was adjusted within 40-100 psi; impurities greater than 250 kD were removed and liquid was concentrated to 50-100 fold. In this example, the concentration multiple was 50 folds.

The concentrated solution was subjected gradient ultracentrifugation, specifically as follows:
10,000 g were centrifuged for 30 min at 4°C; supernatant was retained and centrifuged for 90 min at 4°C; precipitate was retained and added to 20 ml D-PBS, and then centrifuged for 90 min at 100,000 g and 4°C; the centrifuged precipitate was retained to obtain the isolated exosome; then centrifuged precipitate was dissolved with 1 ml D-PBS and mixed well, and stored at -80°C further use.

### Effect Example I:

Comparison of the human umbilical cord mesenchymal stem cell and an exosome thereof before and after being pretreated in biological characteristics
In the description, *in vitro* secretion of the cytokine was detected to further describe the influences of the cytokine composition on the biological characteristics of the human umbilical cord mesenchymal stem cell. The detection on *in vitro* secretion of the cytokine is mainly to survey the concentration of the cytokine in the cultural supernatant of the ordinary human umbilical cord mesenchymal stem cell, the human umbilical cord mesenchymal stem cell pretreated at different cytokine concentrations, and the human umbilical cord mesenchymal stem cell pretreated with different cytokine compositions (the combination type includes: the cytokine composition 1, the cytokine composition 2, and the cytokine composition 3) at culture conditions *in vitro* within the same culture time.

Specifically, when the degree of cell fusion was up to more than 80%, the complete medium containing the corresponding concentration of cytokine or different combinations of cytokines was added to stimulate the mesenchymal stem cell for 10-24 h; cultural supernatant was collected and the expression of the mesenchymal stem cell was detected; meanwhile, the above complete medium was replaced with a basal medium for starvation treatment for 24 h, and the expression of the cytokine in the exosome was detected.

The exosome of the human umbilical cord mesenchymal stem cell before and after being pretreated is obtained in the following way: the pretreated mesenchymal stem cell was subjected to starvation treatment after replacing into the basal medium, and then cultural supernatant was collected and subjected to gradient centrifugation, to obtain the exosome of the concentrated mesenchymal stem cell having enhanced anti-SLE activity, and the exosome was tested and assayed by a nanometer particle size analyzer and western blot. The "pretreatment time" refers to time for contact culture.

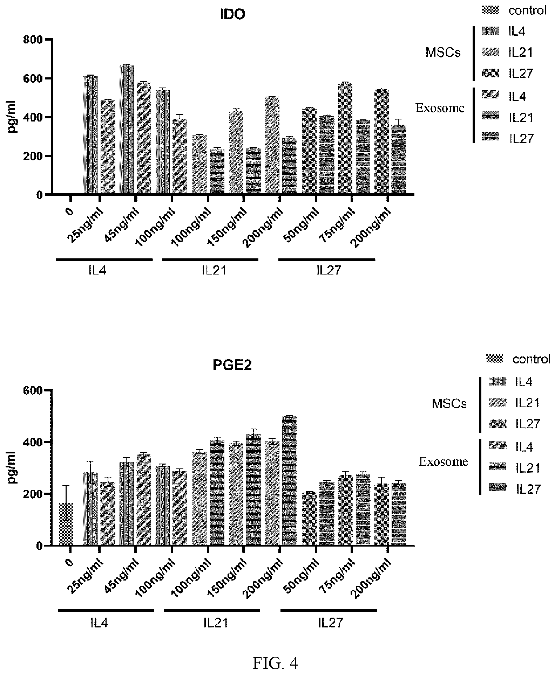

Results of FIGS. 3 and 4 show that as can be seen from the expression quantity of the proteins IDO, PGE2, and VEGF assayed by ELISA, the concentrations of the IDO, PGE2, and VEGF obviously increased in the cultural supernatant and exosome of the human umbilical cord mesenchymal stem cell after being pretreated.

FIG. 4 shows that when the human umbilical cord mesenchymal stem cell and the exosome thereof was pretreated by a single cytokine at different concentrations for expression, the IL4 preferably had a concentration of 45 ng/ml, the IL21 preferably had a concentration of 200 ng/ml, and the IL27 preferably had a concentration of 75 ng/ml.

### Effect Example II:

*In vitro* immunosuppression test on the human umbilical cord mesenchymal stem cell and an exosome thereof before and after being pretreated

*In vitro* co-culture of the mesenchymal stem cell and a human peripheral blood mononuclear cell (PBMC) is an important test method to detect the *in vitro* immunosuppression capability of the mesenchymal stem cell. In the present invention, human PBMC was labeled with CFSE and simulated by monoclonal antibodies CD3 and CD28, and then co-cultured with an ordinary human umbilical cord mesenchymal stem cell or the exosome thereof, the human umbilical cord mesenchymal stem cell after being pretreated or the exosome thereof (the combination type includes: the cytokine composition 1, the cytokine composition 2, and the cytokine composition 3) for 72-80 h; the proliferation of human PBMC was assayed by flow cytometry.

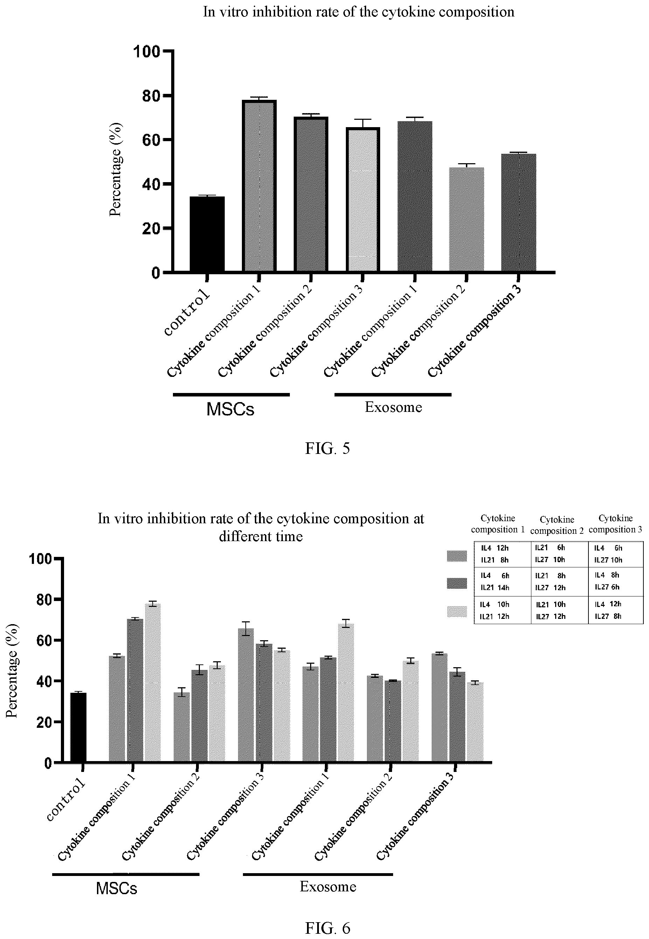

Results of the FIGS. 5-7 show that after being co-cultured for 80 h, the human umbilical cord mesenchymal stem cell after being pretreated by the cytokine composition or the exosome thereof has an immunosuppression effect obviously superior to that the ordinary human umbilical cord mesenchymal stem cell or the exosome thereof. As can be seen from the results of the flow cytometry, the proliferation ratio of the CD3+ total T cells co-cultured by the human umbilical cord mesenchymal stem cell pretreated by the cytokine composition 1, the cytokine composition 2, or the cytokine composition 3 or the exosome thereof is obviously reduced, and the proportion of the proliferation decline is obviously higher than that of the CD3+ total T cells co-cultured by the ordinary human umbilical cord mesenchymal stem cell or the exosome thereof. Based on the statistics on percentage inhibition, the human umbilical cord mesenchymal stem cell after being pretreated by the cytokine composition or the exosome thereof has a good inhibitory effect on the CD3+ total T cells during the co-culture; and the inhibition ratio is obviously higher than that of the ordinary human umbilical cord mesenchymal stem cell or the exosome thereof.

Results of FIG. 6 shows that during the pretreatment, in the cytokine composition 1, the cell was pretreated with the IL4 for 10 h and pretreated with the IL21 for 12 h; in the cytokine composition 2, the cell was pretreated with the IL21 for 10 h and pretreated with the IL27 for 12 h; and in the cytokine composition 3, the cell was pretreated with the IL4 for 6 h and pretreated with the IL27 for 10 h.

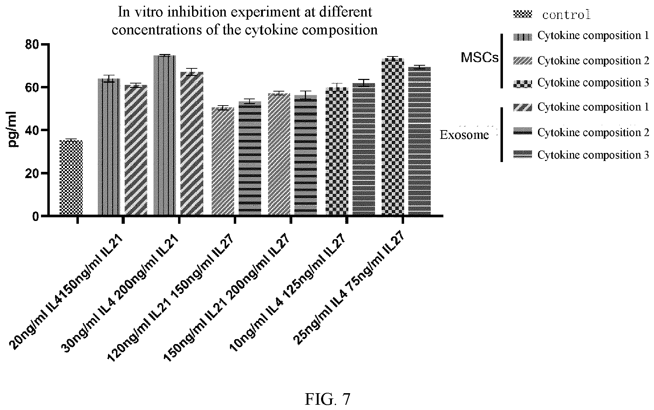

Results of FIGS. 7-8 show that as can be seen the *in vitro* immunosuppression test and the SLE therapy model test, the cytokine composition 1 preferably has 30 ng/ml IL4 and 200 ng/ml IL21; the cytokine composition 2 preferably has 150 ng/ml IL21 and 200 ng/ml IL27; and the cytokine composition 3 preferably has 25 ng/ml IL4 and 75 ng/ml IL27.

### Effect Example III:

SLE mice therapy model of the human umbilical cord mesenchymal stem cell pretreated with a cytokine composition and an exosome thereof
MRL/lpr mice, as SLE mouse models, would spontaneously suffer an onset of illness after being fed to 12-week old in a laboratory; it began to detect the changes of the cytokines (IL4, IL-18, and IL27) and concentrations of the anti-nuclear antibody (ANA), and anti-ds DNA antibody (dsDNA) in the SLE mice serum from the 10th week. In the present invention, the immunosuppression functions of the ordinary human umbilical cord mesenchymal stem cell or the exosome thereof, and the human umbilical cord mesenchymal stem cell after being pretreated or the exosome thereof (the combination type includes: the cytokine composition 1, the cytokine composition 2, and the cytokine composition 3) were applied to the treatment of SLE.

The "SLE therapy model test" is as follows: a MRL/lpr-gene mouse (the mouse would appear the disease features similar to the clinical SLE at 12-week age and thus is a good SLE research model) was injected with 1×10⁶ human umbilical cord mesenchymal stem cells before and after being pretreated via the caudal vein, or injected with the exosome (total protein content: 200 µg) of the human umbilical cord mesenchymal stem cells before and after being pretreated via the caudal vein at 12-week age. After molding, the mouse was killed on the 14th day; its internal organs and tissues were taken and subjected to pathological section, and stained by H&E together with the control group; and then therapeutic effects were judged.

As shown in FIGS. 2, and 5-8, the results show that the ordinary human umbilical cord mesenchymal stem cell and the exosome thereof has certain therapeutic effect, indicating that the ordinary human umbilical cord mesenchymal stem cell and the exosome thereof may exert the corresponding immunosuppression effect after being stimulated by inflammatory factors *in vivo;* however, the ordinary human umbilical cord mesenchymal stem cell is not pretreated with a specific cytokine and thus, may not come into play immediately upon entry into the body and has a limited therapeutic effect. Moreover, the exosome released by the non-treated human umbilical cord mesenchymal stem cell also shows the same biological characteristics, indicating that the non-pretreated human umbilical cord mesenchymal stem cell may have a limited immune modulating effect. However, in the SLE models, the human umbilical cord mesenchymal stem cell after being pretreated by the cytokine composition 1, the cytokine composition 2, or the cytokine composition 3 or the exosome thereof shows an immune modulating effect superior to that of the ordinary human umbilical cord mesenchymal stem cell or the exosome thereof.

Therefore, the above result accords with the conclusion of the *in vitro* experiment in the description. The cytokine composition provided by the present invention has enhanced effects on the human umbilical cord mesenchymal stem cell or the exosome thereof in the treatment of SLE.

FIGS. 2, and 5-8 show that the mesenchymal stem cell cultured by the cytokine composition and the exosome thereof provided by the present invention has good anti-inflammatory activity against SLE. By experiment validation, compared with the existing ordinary mesenchymal stem cell or the exosome thereof, the cytokine composition treated by at least two of cytokines IL4, IL21, and IL27 and the exosome thereof have markedly improved therapeutic effect against SLE, and may effectively reduce the secretion levels of dsDNA and ANA in the animal model.

The "anti-SLE activity" refers that the mesenchymal stem cell treated by at least two of cytokines IL4, IL21, and IL27 and the exosome thereof have better immunosuppression functions against T/B lymphocytes.

In the present invention, the "anti-SLE activity" is mainly achieved by the following mechanism: the mesenchymal stem cell is *in vitro* pretreated by the corresponding cytokine composition in advance to contact the cytokine that comes into play mostly under disease microenvironment ahead of time such that the mesenchymal stem cell is activated during culture *in vitro.* Such a mechanism shortens the activation time upon entry into the body, and enhance the functionalities of the cell to special microenvironment, thus improving the antagonistic activity and therapeutic effect to the disease.

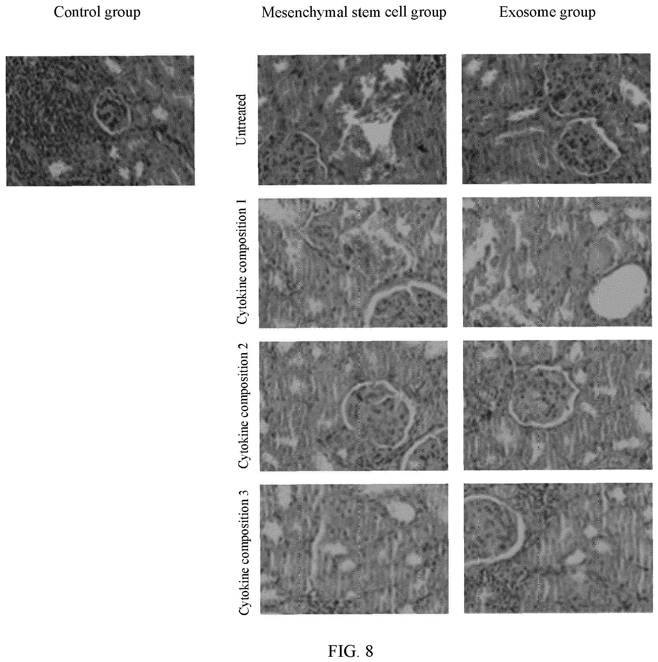

Results of FIG. 8 show that the mesenchymal stem cell after being pretreated in the description or the exosome thereof achieves obvious therapeutic effects after the treatment.

The design of the present invention is focused on the follows: the mesenchymal stem cell obtained after being pretreated by the cytokine composition and the exosome thereof provided by the present invention may exert a stronger immune modulating effect and may be used for the treatment of immune diseases better. Meanwhile, the present invention lays a foundation for providing a personalized cell therapeutic regimen on the basis of different states of a disease. The present invention provides a set of feasible clinical therapeutic regimens for stem cells for precise treatment, and may achieve targeted precise treatment based on the order of severity of the SLE patients, thereby effectively improving the therapeutic effect and reducing clinical symptoms of the disease.

What is described above are merely preferred embodiments of the present invention, but are not construed as limiting the technical scope of the present invention. Therefore, any minor amendment, equivalent change and modification made to the above embodiments based on the technical essence of the present invention still fall within the technical scope of the present invention.

## Claims

1. A mesenchymal stem cell treated by at least two of cytokines IL4, IL21, and IL27, and an exosome thereof, **characterized in that** a mesenchymal stem cell is pretreated by a cytokine composition to obtain the mesenchymal stem cell or the exosome thereof; the cytokine composition comprises at least two of cytokines IL4, IL21, and IL27; the mesenchymal stem cell is obtained in the following way of pretreatment: treating and stimulating the mesenchymal stem cell by stages with a complete medium containing the cytokine composition by a large-scale bioreactor to obtain a capacitated mesenchymal stem cell; the exosome is obtained in the following way of pretreatment: treating and stimulating the mesenchymal stem cell by stages with a complete medium containing the cytokine composition by a large-scale bioreactor, replacing with a basal medium for starvation treatment, collecting a cultural supernatant, and conducting gradient ultracentrifugation to obtain the exosome.

2. The mesenchymal stem cell treated by at least two of cytokines IL4, IL21, and IL27, and an exosome thereof according to claim 1, **characterized in that** the way of treating and stimulating by stages is as follows: adding a complete medium containing different cytokine compositions having corresponding concentrations to the large-scale bioreactor by stages, to capacitate and stimulate the mesenchymal stem cell; and the complete medium is a pooled serum substitute for any basal medium for culturing a mammalian cell.

3. The mesenchymal stem cell treated by at least two of cytokines IL4, IL21, and IL27, and an exosome thereof according to claim 1, **characterized in that** the three cytokines IL4, IL21, and IL27 have concentration ranges of IL4: 25-150 ng/ml, IL21: 100-300 ng/ml, and IL27: 50-250 ng/ml, respectively.

4. The mesenchymal stem cell treated by at least two of cytokines IL4, IL21, and IL27, and an exosome thereof according to claim 1, **characterized in that** the cytokine composition comprises the IL4 and the IL21, or the IL21 and the IL27, or the IL4 and the IL27.

5. The mesenchymal stem cell treated by at least two of cytokines IL4, IL21, and IL27, and an exosome thereof according to claim 4, **characterized in that** the cytokine composition comprises the IL4 having a concentration of 30 ng/ml and the IL21 having a concentration of 200 ng/ml, or the IL21 having a concentration of 150 ng/ml and the IL27 having a concentration of 200 ng/ml, or the IL4 having a concentration of 25 ng/ml and the IL27 having a concentration of 75 ng/ml.

6. The mesenchymal stem cell treated by at least two of cytokines IL4, IL21, and IL27, and an exosome thereof according to claim 4, **characterized in that** contact culture is required in the pretreatment process of the mesenchymal stem cell by the cytokine composition for 16-36 h; the mesenchymal stem cell is pretreated with the IL4 in the cytokine composition containing IL4 and IL21 for 10 h and pretreated with the IL21 in the cytokine composition containing IL4 and IL21 for 12 h; the mesenchymal stem cell is pretreated with the IL21 in the cytokine composition containing IL21 and IL27 for 10 h and pretreated with the IL27 in the cytokine composition containing IL21 and IL27 for 12 h; and the mesenchymal stem cell is pretreated with the IL4 in the cytokine composition containing IL4 and IL27 for 6 h and pretreated with the IL27 in the cytokine composition containing IL4 and IL27 for 10 h.

7. The mesenchymal stem cell treated by at least two of cytokines IL4, IL21, and IL27, and an exosome thereof according to claim 1, **characterized in that** the basal medium is used for starvation treatment for 24 h.

8. The mesenchymal stem cell treated by at least two of cytokines IL4, IL21, and IL27, and an exosome thereof according to claim 1, **characterized in that** the mesenchymal stem cell not pretreated is derived from at least one of the group consisting of bone marrow, fat, placenta, umbilical cord, and dental pulp in a human tissue; and the exosome is secreted from the mesenchymal stem cell.

9. The mesenchymal stem cell treated by at least two of cytokines IL4, IL21, and IL27, and an exosome thereof according to claim 1, **characterized by** having an enhanced anti-systemic lupus erythematosus (SLE) curative activity.

10. Use of the mesenchymal stem cell treated by at least two of cytokines IL4, IL21, and IL27, and an exosome thereof according to any one of claims 1-9, **characterized by** for use in the preparation of a medicament for treating a systemic and immune inflammation caused by SLE.
